# EUROPÄISCHE PATENTANMELDUNG

(11) **EP 2 497 760 A1**
(43) Veröffentlichungstag der Anmeldung: **12.09.2012**
(21) Anmeldenummer: 12155417.4
(22) Anmeldetag: 14.02.2012
(51) Int. Cl.: C07C 37/16, H01B 3/30

(54) **Verfahren zur Herstellung von alkylierten Hydroxyaromaten in Mikroreaktoren**

(30) Priorität: 08.03.2011 DE 102011005228
(71) Anmelder: Elantas GmbH, 46483 Wesel (DE)
(72) Erfinder: Bleifuß, Oliver, 29640 Schneverdingen (DE); Rost, Simon, 21514 Büchen (DE); Lienert, Klaus-Wilhelm, 22763 Hamburg (DE); Moritz, Hans-Ulrich, 21227 Bendestorf (DE)
(74) Vertreter: Schuck, Alexander

(57) **Zusammenfassung**

Es wird ein Verfahren zur Herstellung von Hydroxyaromaten durch heterogene, katalytische Umsetzung von Hydroxyaromaten mit C₁-C₄-Alkanolen in einem Mikroreaktor (10) vorgeschlagen, das die Schritte umfasst:
a) Einleiten des Hydroxyaromaten und mindestens eines C₁-C₄-Alkanols als Edukte in mindestens eine Einlassöffnung (22) des Mikroreaktors (10) umfassend mindestens eine Mikroreaktoreinheit (18),
b) Durchleiten der Edukte durch mindestens eine Mikroreaktoreinheit (18) des Mikroreaktors (10), die eine Mehrzahl von Mikrokanälen (28) umfasst, wobei die Mikrokanäle (28) eine laterale Ausdehnung von weniger als 1 mm aufweisen und in den Mikrokanälen (28) ein heterogener Katalysator zur Umsetzung der Edukte eingebracht ist,
c) Ausleiten der hergestellten Hydroxyaromate durch mindestens eine Auslassöffnung (24) des Mikroreaktors (10).

## Beschreibung

Die Erfindung betrifft ein Verfahren zur Herstellung von alkylierten Hydroxyaromaten durch heterogene, katalytische Umsetzung von Hydroxyaromaten mit C₁-C₄-Alkanolen. Die Erfindung betrifft ferner die Verwendung der mittels des erfindungsgemäßen Verfahrens hergestellten alkylierten Hydroxyaromaten als Lösungsmittel in Drahtlack-Zusammensetzungen.

Phenol und dessen Alkylderivate, wie beispielsweise Kresole, Xylenole und Anisole, sind insbesondere für die Herstellung von Kunstharzen und Lacken von Bedeutung. In der Elektrotechnik werden diese Verbindungen regelmäßig für Lacke verwendet, die insbesondere zur elektrischen Isolierung von Kupfer- und Aluminiumdrähten geeignet sind. Diese Drahtlacke müssen daher viele Anforderungen gleichzeitig erfüllen. Sie sollten eine hohe chemische und thermische Beständigkeit, eine gute Haftung auf Drähten und eine besonders gute mechanische Festigkeit aufweisen. Aufgrund der hohen Materialanforderungen bedarf es daher einer stetigen Verbesserung der Materialeigenschaften der eingesetzten Drahtlacke.

Die Herstellung von alkylierten Hydroxyaromaten, wie sie als Lösungsmittel für Drahtlacke verwendet werden, erfolgt üblicherweise in konventionellen Reaktortypen wie Rohr(bündel)- oder Wirbelbettreaktoren. Bei dieser Art von Reaktoren liegt der Katalysator typischerweise im Fest-, Fließ- oder Wirbelbett vor. Je nach verwendetem Katalysator erfolgt die Herstellung von alkylierten Hydroxyaromaten in konventionellen Reaktoren bei hohen Temperaturen oder unter hohem Druck, um eine bessere Raum-Zeit-Ausbeute zu erreichen.

GB 1 065 337 beispielsweise beschreibt ein Alkylierungsverfahren mittels Alkanol, vorzugsweise Methanol oder Ethanol, wobei die Reaktionskomponenten im Molverhältnis 0,6:1 bis 0,8:1 in der Gasphase über einen gegebenenfalls aktivierten Aluminiumoxidkatalysator geleitet werden. Als Beispiel ist die Gewinnung von o-Kresol beschrieben, das in Ausbeuten von durchschnittlich 27,3 Massen-% erhalten wird unter gleichzeitiger Bildung von 12,4 Massen-% 2,6-Dimethylphenol und 5,0 Massen-% anderer Phenole.

In DE 1 668 880 wird ein Gemisch von Phenol und Methanol (10 Massen-% Methanol) bei 250 bis 350 °C und einer Flüssigkeitsraumgeschwindigkeit (liquid hourly space velocity, LHSV) von 0.5 (h⁻¹) über einen üblichen Aluminiumoxid-Kontakt mit einer spezifischen Oberfläche (BET) von 240 m²/g geleitet, wobei ein Produktgemisch von 22 Massen-% o-Kresol, 5 Massen-% 2,6-Dimethylphenol und Spuren von Anisol erhalten wurde. Versuche ergaben, dass neben diesen Produkten noch weitere Methylierungsprodukte von Phenol, wie m-, p-Kresol, Di- und Trimethylphenole anfallen, deren Menge ca. 2 Massen-% beträgt. Verwendet wird in diesem Fall ein Rohrreaktor mit einem Außendurchmesser von 20 mm und einer Länge von 1 m.

GB 1 034 500 beschreibt die Alkylierungsreaktion in einem Festbettreaktor unter Verwendung von Magnesiumoxid als Katalysator. Hierbei sind jedoch hohe Reaktionstemperaturen von 475 bis 600°C erforderlich, bei denen schon eine starke Zersetzung der Ausgangsmaterialien, z.B. des Phenols und Methanols, eintritt. Ein weiterer Nachteil liegt in der rasch nachlassenden Aktivität und der geringen Standzeit des Katalysators.

Die Verwendung von Mischkatalysatoren wie Vanadium/Eisenoxid oder von Ceroxid/Manganoxid für die Alkylierung von Phenolen wird in DE 2161 252 und in DE 2 127 083 vorgeschlagen. Diese Katalysatortypen zeigen jedoch eine relativ geringe Reaktivität, so dass auch für die Erzeugung von monoalkylierten Produkten ein hoher Überschuss an Alkylierungsmittel (z.B. Methanol) erforderlich ist und außerdem relativ hohe Temperaturen und relativ niedrige Durchsätze angewandt werden müssen. So erfolgt nach der DE 2 127 083 die o-Kresol-Synthese bei 400 °C und einer LHSV von 0,3, wobei das Verhältnis von Nebenprodukten (z.B. 2,6-Dimethylphenol) zu o-Kresol 0,38 beträgt. Hierbei werden herkömmliche Rohrreaktoren für die Gasphasenreaktionen verwendet.

In US 3,290,389 und US 3,367,981 werden Verfahren beschrieben, bei denen Aluminum-Katalysatoren zur Alkylierung von Phenolen verwendet werden. Sie beschreiben, dass eine Substitution im Wesentlichen in der ortho-Position stattfindet. Die in diesen Schriften beschriebenen Reaktionen werden bei Temperaturen oberhalb von 250 °C in Druckgefäßen durchgeführt. CN 101514145 beschreibt ein weiteres Syntheseverfahren für o-Kresol. Dieses Verfahren ist durch folgende Schritte charakterisiert: Mischen von Phenol und Methanol in einem Masseverhältnis 1:(0,5-4), Fördern der Mischung in einen Rohrreaktor, der den Katalysator enthält, Einleiten eines inerten Trägergases in den Reaktor und abschließend Kondensation und Trennung der Reaktionsprodukte. Die Reaktionen werden bei Normaldruck und Temperaturen von 280 bis 450 °C durchgeführt.

Bekannt ist auch die Gewinnung von Kresolen als Nebenprodukte bei der Synthese von Dimethylphenolen und die anschließende Isolierung des o-Kresols durch zusätzliche Reinigungsschritte. Verfahren zur Erzeugung von o-Kresol sind in Frank/Stadelhofer, INDUSTRIELLE AROMATENCHEMIE: Rohstoffe, Verfahren, Produkte, Springer Verlag, Berlin 1987 beschrieben.

Die bekannten Verfahren zur Herstellung von alkylierten Hydroxyaromaten werden vorwiegend in konventionellen Reaktoren durchgeführt. Bei diesen Reaktoren ist es aufgrund der Abmessungen schwierig, eine einheitliche Temperaturverteilung über das Reaktorvolumen einzustellen. Dies erhöht das Risiko, den Katalysator einer übermäßigen thermischen Belastung auszusetzen, was die Katalysatorleistung und die Katalysatorlebensdauer beeinflusst.

Weiterhin gestaltet sich die Herstellung von alkylierten Hydroxyaromaten in einem industriellen Maßstab schwierig. Denn beim Übertragen chemischer Verfahren in den großtechnischen Produktionsmaßstab besteht das grundsätzliche Problem, dass die Abmessungen der Produktionsanlagen um mehrere Größenordnungen größer sind als die im Labormaßstab zur Entwicklung der Verfahren verwendeten Geräte. Aufgrund der großtechnisch benötigten Produktionsvolumina haben konventionelle chemische Reaktoren meistens Abmessungen, die in der Größe zwischen wenigen Zentimetern bis zu mehreren Metern liegen. Daher können die im Labormaßstab gewonnenen Erkenntnisse über die Verfahrensführung nicht direkt in den großtechnischen Maßstab übernommen werden. Bereits beim Vermischen von Flüssigkeiten ist ein Rührwerk primär dafür nötig, den Stofftransport dadurch zu vergrößern, dass die Abstände zwischen Gebieten unterschiedlicher Konzentration verringert werden.

Aus den unterschiedlichen Dimensionen der Reaktoren ergibt sich auch das sogenannte Scale-up-Problem. Eine chemische Reaktion, die im Labormaßstab optimiert wurde, wird danach nicht sofort auf die Produktionsanlage übertragen, sondern zunächst in einer Pilot-Anlage mit Dimensionen zwischen dem Labor- und dem Produktionsmaßstab getestet, bevor sie schließlich in der großtechnischen Produktion eingesetzt wird. Problematisch ist hierbei, dass jede Stufe dieser Verfahrensentwicklung eines eigenen Optimierungszyklus bedarf, wobei jeder dieser Zyklen additiv in die für die Prozesseinführung benötigte Entwicklungsdauer eingeht.

Trotz des umfangreichen Standes der Technik auf dem Gebiet der Herstellung von alkylierten Hydroxyaromaten besteht ständiger Optimierungsbedarf im Hinblick auf höhere Raum-Zeit-Ausbeuten, um die chemischen Prozesse effizienter zu gestalten. Demnach bestand die Aufgabe der Erfindung darin, ein Verfahren zur Herstellung von alkylierten Hydroxyaromate bereitzustellen, das eine höhere Raum-Zeit-Ausbeute aufweist. Auch soll das Verfahren ein Produkt zur Verfügung stellen, das insbesondere für Drahtlacke eingesetzt werden kann.

Gelöst wird diese Aufgabe durch ein Verfahren zur Herstellung von Hydroxyaromaten durch heterogene, katalytische Umsetzung von Hydroxyaromaten mit C₁-C₄-Alkanolen in einem Mikroreaktor umfassend die Schritte:
a) Einleiten des Hydroxyaromaten und mindestens einer Verbindung, ausgewählt aus der Gruppe bestehend aus C₁-C₄-Alkanolen, als Edukte in mindestens eine Einlassöffnung des Mikroreaktors umfassend mindestens eine Mikroreaktoreinheit,
b) Durchleiten der Edukte durch mindestens eine Mikroreaktoreinheit des Mikroreaktors, die eine Mehrzahl von Mikrokanälen umfasst, wobei die Mikrokanäle eine laterale Ausdehnung von weniger als 1 mm aufweisen und in den Mikrokanälen ein heterogener Katalysator zur Umsetzung der Edukte eingebracht ist,
c) Ausleiten der hergestellten Hydroxyaromate durch mindestens eine Auslassöffnung des Mikroreaktors.

Ein Hydroxyaromat im Sinne der vorliegenden Erfindung weist mindestens einen aromatischen Ring auf, an dem mindestens eine Hydroxylgruppe gebunden ist. Entsprechend weist ein alkylierter Hydroxyaromat einen aromatischen Ring auf, an dem zusätzlich mindestens eine Alkylgruppe gebunden ist.

Ein Mikroreaktor, wie er in dem erfindungsgemäßen Verfahren eingesetzt wird, umfasst mindestens eine Einlassöffnung, die über mindestens eine Mikroreaktoreinheit mit mindestens einer Auslassöffnung verbunden ist. Dabei umfasst die mindestens eine Mikroreaktoreinheit des Mikroreaktors, in der die chemische Reaktion stattfindet, eine Mehrzahl von Mikrokanälen mit einer lateralen Ausdehnung von weniger als 1 mm. Hierbei bezeichnet die laterale Ausdehnung die Abmessungen eines Mikrokanals in einer Ebene senkrecht zu der Mikrokanalachse. Weiterhin ist in die Mehrzahl von Mikrokanälen ein heterogener Katalysator eingebracht.

Das erfindungsgemäße Verfahren nutzt zur Herstellung von alkylierten Hydroxyaromaten in vorteilhafter Weise einen solchen Mikroreaktor. Denn in dem Mikroreaktor umfassend eine Mikroreaktoreinheit wird ein Stoffstrom durch eine Mehrzahl von Mikrokanälen geleitet. Mit einer lateralen Ausdehnung von weniger als 1 mm weisen die Mikrokanäle ein Volumen zu Oberfläche-Verhältnis auf, das eine bessere Kontrolle von Prozessparametern erlaubt, als dies bei konventionellen Reaktoren, wie zum Beispiel Rohrbündelreaktoren, der Fall ist. So wird aufgrund der lateralen Ausdehnung eines jeden Mikrokanals von weniger als 1 mm und demzufolge dem geringen Volumenstrom in jedem Mikrokanal ein optimaler Stoff- und Wärmetransport erreicht. Hierdurch kann insbesondere die Temperatur in jedem Mikrokanal bzw. die Temperatur des Stoffstroms in jedem Mikrokanal kontrolliert werden. Dies ist besonders vorteilhaft für das erfindungsgemäße Verfahren, das auf heterogener Katalyse beruht und den heterogenen Katalysator in der Mehrzahl von Mikrokanälen vorsieht, da einer Zersetzung des Katalysators durch die Temperaturkontrolle vorgebeugt werden kann.

Zusätzlich werden durch die kleinen Abmessungen der Mikrokanäle lokale Unterschiede der Konzentration und Temperatur des Stoffstroms auf ein minimales Maß verringert. Dadurch lässt sich das erfindungsgemäße Verfahren auf optimale Randbedingungen einstellen, so dass die Umsatzraten an alkylierten Hydroxyaromaten bei gleicher Verweilzeit wie in konventionellen Reaktoren vergrößert werden können. Alternativ können bei verringerter Verweilzeit ähnliche Umsatzraten wie in konventionellen Reaktoren erreicht werden. Darüber hinaus werden auch die Reinheit und die Ausbeute an alkylierten Hydroxyaromaten durch die einstellbaren Prozessbedingungen optimiert. Auf diese Weise stellt das erfindungsgemäße Verfahren alkylierte Hydroxyaromaten zur Verfügung, die überraschenderweise hervorragende Lösungsmitteleigenschaften für Drahtlacke aufweisen.

Weiterhin werden durch die Verwendung des Mikroreaktors in dem erfindungsgemäßen Verfahren zur Herstellung von alkylierten Hydroxyaromaten Probleme vermieden, die beim Übertragen des Verfahrens vom Labormaßstab in die industrielle Großproduktion auftreten. So ist der Volumenstrom eines einzelnen Mikrokanals aufgrund der lateralen Abmessung gering. Für eine Mehrzahl von Mikrokanälen addiert sich jedoch der Volumenstrom der einzelnen Mikrokanäle. Auf diese Weise kann mit einem Mikroreaktor trotz der kleinen Abmessungen der Mikrokanäle insgesamt ein großer Volumenstrom erreicht werden, und eine Übertragung vom Labormaßstab zur industriellen Großproduktion kann durch einfache Vervielfältigung der Mikrokanäle erfolgen.

### Mikroreaktor

Der in dem erfindungsgemäßen Verfahren verwendete Mikroreaktor kann neben der mindestens einen Mikroreaktoreinheit, in der die chemische Reaktion stattfindet, weitere funktionelle Einheiten umfassen, die zusätzliche Funktionen in der chemischen Prozessführung ausführen. Die Ausgestaltung solcher funktionellen Einheiten ist beispielsweise in US 5,534,328, US 5,811,062 oder US 5,690,763 beschrieben. So kann mindestens eine Einheit zum Mischen der Edukte zwischen der Einlassöffnung des Mikroreaktors und der mindestens einen Mikroreaktoreinheit, in der die chemische Reaktion stattfindet, vorgesehen werden. Alternativ kann der Einlassöffnung des Mikroreaktors ein externer Mischer vorgeschaltet sein, der die Edukte bereits vor dem Zuführen in den Mikroreaktor mischt. Die Zuführung der Edukte oder des Eduktgemisches erfolgt dabei im Allgemeinen durch eine Dosierpumpe, die den Stoffstrom und den Druck in dem Mikroreaktor regelt.

Weiterhin kann mindestens eine Einheit des Mikroreaktors als Wärmetauscher ausgestaltet sein, um Wärme einer benachbarten Mikroreaktoreinheit zuzuführen oder von einer benachbarten Mikroreaktoreinheit abzuführen. Auf diese Weise kann die Temperatur in einzelnen Mikroreaktoreinheiten je nach ihrer funktionellen Bestimmung in der chemischen Prozessführung kontrolliert werden. Gemäß einer Ausführungsform wird die Temperatur in der mindestens einen Mikroreaktoreinheit, in der die chemische Reaktion stattfindet, durch benachbarte Wärmetauscher-Einheiten kontrolliert. Gemäß einer bevorzugten Ausführungsform wird die Temperatur in der mindestens einen Mikroreaktoreinheit, in der die chemische Reaktion stattfindet, in einfacher Weise durch externe Heizelemente, beispielsweise durch Heizmanschetten oder Heizpatronen kontrolliert. Die Temperaturmessung erfolgt dabei regelmäßig mittels Temperatursensoren, die beispielsweise an einer Mikroreaktoreinheit und an einem Auslass des Mikroreaktors angebracht sein können.

Zur Ausbildung der Mikrokanäle in mindestens einer Mikroreaktoreinheit werden, wie beispielsweise in US 5,534,328 beschrieben, mikrostrukturierte Platten eingesetzt. Diese sind vorzugsweise aus einem Material mit hoher Wärmeleitfähigkeit, wie zum Beispiel Stahl oder Silizium gefertigt. Zum Ausbilden der mikrostrukturierten Platten wird eine Mehrzahl von Vertiefungen in die Platten eingebracht, die je nach gewünschtem Profil der Mikrokanäle halbrund, oval, trapezförmig oder rechteckig ausgestaltet sein können. Diese Vertiefungen können sowohl einseitig als auch beidseitig mittels bekannter Verfahren wie Fräsen, Nassätzen oder Diamantschneiden in die Platten eingebracht werden. Zum Ausbilden einer Mehrzahl von Mikrokanälen können die mikrostrukturierten Platten so gegeneinander ausgerichtet gestapelt werden, dass die Mehrzahl von komplementären Vertiefungen eine Mehrzahl von Mikrokanälen ausbildet.

Die mindestens eine Mikroreaktoreinheit wird demnach aus mindestens zwei aufeinanderliegenden Platten, welche an der Oberfläche die Mikrokanäle bildenden Vertiefungen aufweisen, gebildet. Vorzugsweise wird eine Mikroreaktoreinheit aus 2 bis 10 aufeinanderliegenden Platten gebildet. Die Struktur der in die mikrostrukturierten Platten eingebrachten Vertiefungen bestimmt somit die dreidimensionale Geometrie der Mehrzahl von Mikrokanälen. Diese Mikrokanäle weisen je nach Profil der Vertiefungen eine maximale laterale Ausdehnung von weniger als 1 mm, vorzugsweise zwischen 0,1 und 0,8 mm, auf. Besonders bevorzugt ist eine je nach Profil der Vertiefungen maximale laterale Ausdehnung zwischen 0,4 und 0,6 mm. Hierbei beziehen sich die Zahlenwerte auf die maximale laterale Ausdehnung senkrecht zur Strömungsrichtung.

Die gestapelten, mikrostrukturierten Platten umfassen im Allgemeinen integrierte Abdichtzonen, die eine flüssigkeitsdichte und gasdichte Verbindung zwischen den mikrostrukturierten Platten und nach außen herbeiführen. Als Dichtmaterial kann beispielsweise Graphit verwendet werden. Die gestapelten Mikroplatten können ferner von einem Gehäuse mit angepasster Größe so umschlossen sein, dass die Abdichtzonen der mikrostrukturierten Platten durch ausreichende Flächenpressung dichtend aufeinander liegen. Der Verbund von zwei oder mehreren Platten bildet demnach eine Mikroreaktoreinheit, die je nach Struktur der Mehrzahl von Mikrokanälen als funktionelle Einheit oder als Mikroreaktoreinheit, in der die chemischen Reaktionen stattfinden, ausgestaltet sein kann. Weiterhin sind in einer Mikroreaktoreinheit Öffnungen vorgesehen, die einen Stofffluss durch die Mikroreaktoreinheit erlauben.

Gemäß einer Ausführungsform des Mikroreaktors, wie er in dem erfindungsgemäßen Verfahren verwendet wird, sind die Mikrokanäle als gerade, von einer Einlassöffnung zu einer Auslassöffnung verlaufende Kanäle ausgebildet. Die Länge der Mikrokanäle beträgt 50 mm bis 150 mm. Diese Ausgestaltung ist einfach in der Herstellung und erfordert keine speziellen Vorkehrungen zur Herstellung der mikrostrukturierten Platten. Weiterhin kann eine einzige Einlassöffnung einer Mikroreaktoreinheit mit gerade verlaufenden Mikrokanälen so ausgestaltet sein, dass alle Mikrokanäle von einem Stoffstrom versorgt werden. Diese Ausgestaltung hat den Vorteil, dass keine weiteren Einlassöffnungen mit externen Versorgungselementen, wie beispielsweise einer Dosierpumpe, einem Mischer oder einem Vorheizer, versehen werden müssen.

### Edukte

Im Folgenden werden die Edukte näher erläutert, die zur Herstellung von alkylierten Hydroxyaromaten gemäß dem erfindungsgemäßen Verfahren dienen.

Zur Durchführung des erfindungsgemäßen Verfahrens können die Edukte in flüssiger Phase, in gasförmiger Phase oder zweiphasig vorliegen. Dabei müssen die einzelnen Edukte dem Mikroreaktor nicht notwendigerweise in derselben Phase zugeführt werden. Die Edukte können ferner direkt innerhalb oder außerhalb des Mikroreaktors gemischt oder vorgeheizt werden. Dies hat den Vorteil, dass sich bei der Zuführung der Edukte in den Mikroreaktor eine erhöhte Flexibilität ergibt und keine weiteren Verfahrensschritte oder zusätzlichen Maßnahmen vorgesehen werden müssen, um beispielsweise beide Edukte in derselben Phase in den Mikroreaktor einzubringen.

Als Hydroxyaromat, welches als Edukt in Gegenwart eines heterogenen Katalysators in den Mikrokanälen mit C₁-C₄-Alkanolen umgesetzt wird, wird üblicherweise mindestens eine Phenol-Verbindung mit mindestens einem aromatisch gebundenen Wasserstoff-Atom eingesetzt. Die mindestens eine Phenol-Verbindung ist vorzugsweise ausgewählt aus der Gruppe bestehend aus Phenol, o-Kresol, m-Kresol, p-Kresol, Xylenolen, Trimethylphenolen, Tetramethylphenolen, Ethylphenolen, Diethylphenolen, Triethylphenolen, *iso*-Propylphenolen, Di-*iso*-propylphenolen, Tri-*iso*-propylphenolen, Tetra-*iso*-propylphenolen, *n*-propylphenolen, Di-*n*-propylphenolen, Tri-*n*-propylphenolen, Tetra-*n*-propylphenolen, *tert*-Butylphenolen, Di-*tert*-butylphenolen, Tri-*tert*-butylphenolen und Tetra-*tert*-butylphenolen. Besonders bevorzugte Phenol-Verbindung ist Phenol.

Die in dem erfindungsgemäßen Verfahren eingesetzten C₁-C₄-Alkanole können einzelne C₁-C₄-Alkanole oder Mischungen daraus umfassen. Das Edukt kann demnach eine Zusammensetzung aus C₁-C₄-Alkanolen gleicher oder unterschiedlicher Kohlenstoffzahl umfassen. Ferner können C₁-C₄-Alkanole mit einheitlicher Anzahl von Kohlenstoffatomen und/oder Mischungen von C₁-C₄-Alkanolen mit unterschiedlicher Anzahl von Kohlenstoffatomen eingesetzt werden. Dazu zählen auch Mischungen verschiedener Isomere von C₁-C₄-Alkanole mit gleicher Kohlenstoffzahl. Bevorzugt sind C₁-C₃-Alkanole. Besonders bevorzugt werden als C₁-C₄-Alkanole Methanol, Ethanol, n-Propanol, Isopropanol oder Mischungen daraus verwendet. Ganz besonders bevorzugt werden als C₁-C₄-Alkanole Methanol, Ethanol oder 2-Propanol eingesetzt.

### Katalysator

Der Mikroreaktor, in dem das erfindungsgemäße Verfahren durchgeführt wird, sieht mindestens eine Mikroreaktoreinheit vor, in der die chemische Reaktion stattfindet. Diese Mikroreaktoreinheit umfasst dazu eine Mehrzahl von Mikrokanälen, die den heterogenen Katalysator zum Umsetzen der Edukte enthalten. Als heterogene Katalysatoren können alle zur Herstellung von alkylierten Hydroxyaromaten geeigneten Katalysatoren, ggf. mit einem geeigneten Trägermaterial, verwendet werden. Bevorzugt werden solche Katalysatoren eingesetzt, die zur Alkylierung von Phenol geeignet sind.

Gemäß einer Ausführungsform wird der heterogene Katalysator als Feststoffkatalysator in die Mehrzahl von Mikrokanälen des Mikroreaktors eingebracht. Dabei kann der heterogene Katalysator in kristalliner, mikro- und/oder mesoporöser Form vorliegen. Im Falle eines Vollkatalysators, der im Wesentlichen aus der aktiven Komponente besteht, kann der heterogene Katalysator vorzugsweise in Form einer Beschichtung an den Wänden der Mikrokanäle ausgebildet sein. Hierbei können je nach Art und Beschaffenheit des heterogenen Katalysators unterschiedliche dem Fachmann bekannte Beschichtungsverfahren wie beispielsweise chemische Gasphasenabscheidung (engl. chemical vapour deposition, CVD) oder physikalische Gasphasenabscheidung (engl. physical vapour deposition, kurz PVD) eingesetzt werden.

Gemäß einer weiteren Ausführungsform umfasst der heterogene Katalysator einen festen Träger, der vorzugsweise eine oder mehrere Komponenten aus der Gruppe bestehend aus anorganischen Oxiden, Kohlenstoff, Polymeren, Ton, Zeolithen oder mesoporösen Feststoffen enthält. Als anorganische Oxide werden beispielsweise Siliziumoxid, Aluminiumoxid, Zinkoxid und Titanoxid eingesetzt. Übliche mesoporöse Feststoffe sind zum Beispiel die kommerzielle erhältlichen MCM-41 oder MCM-48, wobei MCM für Mobile Crystaline Matter steht. Für das erfindungsgemäße Verfahren wird ein geeigneter fester Träger in der Mehrzahl von Mikrokanälen vorgesehen, die in der mindestens einen Mikroreaktoreinheit zur katalytischen Umsetzung enthalten sind. Bei der Wahl eines geeigneten Trägermaterials sind je nach Ausgestaltung des erfindungsgemäßen Verfahrens Aspekte wie beispielsweise die Größe der zur Verfügung gestellten Oberfläche, die Anzahl von Poren oder die thermische Stabilität zu berücksichtigen.

Der heterogene Katalysator kann ferner auf dem Träger eine Aktivkomponente umfassen. In vorteilhafter Weise wird der heterogene Katalysator durch Washcoat-Beschichten der Platten und ggf. Imprägnierung der Washcoat-Beschichtung mit einer Aktiv-Komponente in die Mikrokanäle eingebracht. Vorzugsweise wird ein Trägermaterial aus anorganischen Oxiden bereitgestellt, indem die Mikrokanäle mit einer Washcoat-Beschichtung, vorzugsweise durch Aufbringen einer flüssigen Suspension, beschichtet und anschließend die Washcoat-Beschichtung ggf. mit der aktiven Komponente imprägniert wird. Ein solches Verfahren ist beispielsweise in R. Zapf, C. Becker-Willinger, K. Berresheim, H. Bolz, H. Gnaser, V. Hessel, G. Kolb, P. Löb, A.-K. Pannwitt, A. Ziogas, Trans IchemE, Vol 81, Part A, 2003, 721-729 beschrieben. Dazu wird eine Suspension so auf die mikrostrukturierten Platten aufgetragen, dass ausschließlich die Mikrokanäle beschichtet sind. Diese Beschichtung wird bei Raumtemperatur getrocknet und gegebenenfalls bei Temperaturen zwischen 500 und 600° C kalziniert. Bei der Kalzinierung werden ungewünschte Zusatzstoffe wie zum Beispiel Bindemittel ausgebrannt.

Danach wird die aktive Komponente des heterogenen Katalysators üblicherweise mittels Imprägnierung in die Beschichtung eingebracht. An die Imprägnierung kann sich eine weitere Trocknung bei 50 bis 500 °C, bevorzugt bei 100 bis 200 °C, bei einem Druck unterhalb oder bei Atmosphärendruck (- 1013 mbar) anschließen. Danach kann eine weitere Kalzinierung bei Temperaturen zwischen 400 und 1500 °C, bevorzugt zwischen 500 und 600 °C, folgen. Die Trocknung und ggf. Kalzinierung dienen dazu, den Katalysator zu aktivieren, wobei die Kalzinierung insbesondere bei der Herstellung von Metalloxid-Katalysatoren, wie beispielsweise Aluminiumoxid oder Siliziumoxid, vorteilhaft ist. Auf diese Weise können Katalysatoren hergestellt werden, deren Eigenschaften, wie zum Beispiel die Regenerierbarkeit, die Abriebfestigkeit, die Reproduzierbarkeit der Herstellung, einstellbar sind. Auch kann die Herstellung zu geringen Kosten erfolgen.

Das erfindungsgemäße Verfahren wird bevorzugt bei einer Temperatur von 300 bis 600 °C und bevorzugt von 350 bis 500 °C durchgeführt. Beim Einsatz von Aluminiumoxid als heterogenen Katalysator hat sich eine Temperatur zwischen 400 und 500 °C als besonders vorteilhaft erwiesen. Unter der genannten Temperatur ist die Temperatur in der Mikroreaktoreinheit zu verstehen, bei der die katalytische Umsetzung stattfindet. Auch für die eingesetzten C₁-C₄-Alkanole ergeben sich bevorzugte Temperaturbereiche. Für Methanol zum Beispiel wird das erfindungsgemäße Verfahren bevorzugt bei einer Temperatur von 400 bis 550 °C und besonders bevorzugt 450 bis 500 °C durchgeführt. Bei der Verwendung von Ethanol wird das erfindungsgemäße Verfahren bevorzugt bei einer Temperatur von 350 bis 500 °C und besonders bevorzugt 400 bis 450 °C ausgeführt. Für 2-Propanol liegt die Temperatur zur Durchführung des erfindungsgemäßen Verfahrens bevorzugt zwischen 330 und 430 °C und besonders bevorzugt zwischen 350 und 390 °C.

### Produkt

Im Rahmen des erfindungsgemäßen Verfahrens werden alkylierte Hydroxyaromaten hergestellt, vorzugsweise alkylierte Phenol-Verbindungen. Bevorzugt werden mit dem erfindungsgemäßen Verfahren Kresole, Xylenole, Ethylphenole, Isopropylphenole, Trimethylphenole, Tetramethylphenole, Pentamethylphenole, Diethylphenole, Triethylphenole, Tetraethylphenole, Pentaethylphenole, Di-*iso*-propylphenole, Tri-*iso-*propylphenole, Tetra-*iso*-propylphenole, Penta-*iso*-propylphenole, Ethylmethylphenole, Methylpropylphenole und Ethylpropylphenole hergestellt. Besonders bevorzugt ist die Herstellung von o-Kresol, *m*-Kresol, *p*-Kresol, 2-Ethylphenol, 2,6-Xylenol oder 2-Isopropylphenol.

Durch das erfindungsgemäße Verfahren hergestellten alkylierten Hydroxyaromaten weisen überraschenderweise hervorragende Lösungsmitteleigenschaften auf, welche beispielsweise zu einem klarem Lack, einer stabilen Viskosität und einem konstanten Verlauf führen. Die alkylierten Hydroxyaromaten können in vorteilhafter Weise in Drahtlack-Zusammensetzungen zur Beschichtung von elektrischen und elektronischen Bauteilen und Geräten, insbesondere Kupferdrähten, eingesetzt werden.

### Zeichnungen

Anhand von Zeichnungen wird der Aufbau eines Mikroreaktors zur Durchführung des erfindungsgemäßen Verfahrens näher beschrieben.

Es zeigen:
- Figur 1: eine schematische Darstellung des Mikroreaktors in der Schnittansicht,
- Figur 2: eine schematische Darstellung von mikrostrukturierten Platten, die Mikrokanäle ausbilden.

### Ausführungsform

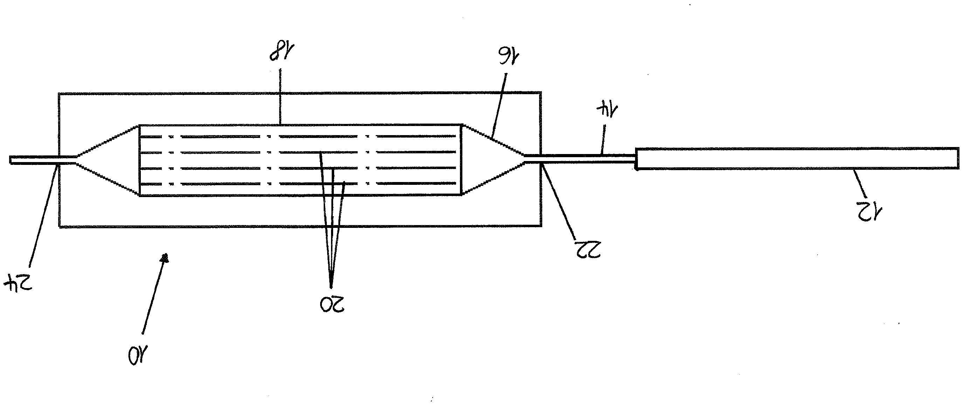

Figur 1 zeigt eine Ausführungsform eines Mikroreaktors 10 in der Schnittansicht. Die Edukte werden mittels eines Verdampfers 12 in ihren dampfförmigen Zustand überführt. Über die Zuleitung 14 wird das Gas zu der Einlassöffnung 22 in dem Mikroreaktor 10 geleitet. Der Verteiler 16 stellt im Bereich vor der Mikroreaktoreinheit 18 Raum zur Verfügung, in dem sich das Gas verteilt. Anschließend wird das Gas durch die Mikroreaktoreinheit 18 geleitet, die in Figur 1 nur schematisch dargestellt ist. Die hergestellten Hydroxyaromaten werden an der Auslassöffnung 24 gewonnen.

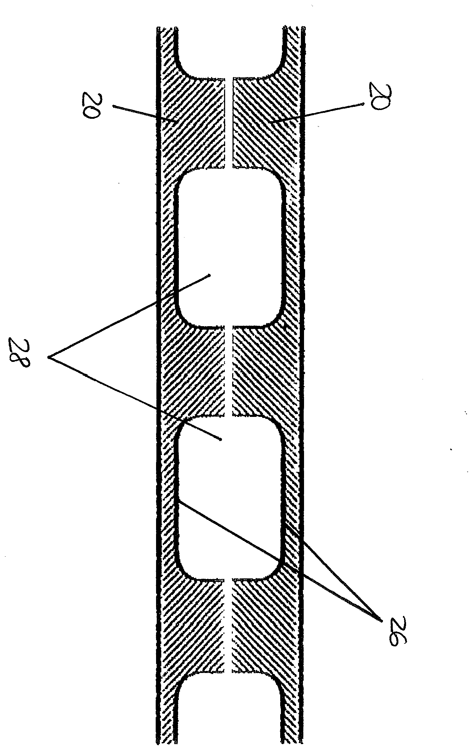

Die Mikroreaktoreinheit 18 der Figur 1 ist aus aufeinanderliegenden, mikrostrukturierten Platten 20 gebildet. Ein Querschnitt zweier aufeinanderliegender, mikrostrukturierter Platten 20 ist beispielhaft in Figur 2 dargestellt. Die beiden Platten 20 weisen jeweils einseitig komplementäre Vertiefungen 26 auf und werden so gegeneinander ausgerichtet, dass eine Mehrzahl von Mikrokanälen 28 ausgebildet wird. Das Profil der Mikrokanäle 28 entspricht einem Rechteck mit abgerundeten Ecken. Wie in Figur 1 angedeutet, sind in einer Mikroreaktoreinheit 18 eine Mehrzahl von gegeneinander ausgerichteten, mikrostrukturierten Platten 20 in einem Gehäuse 30 des Mikroreaktors 10 aufgenommen. Diese bilden von einer Einlassöffnung 22 zu einer Auslassöffnung 24 verlaufende Mikrokanäle 28, in denen die chemische Reaktion stattfindet.

### Beispiele

Die Erfindung wird durch nachstehende Beispiele näher beschrieben.

Im Rahmen der Beispiele wird ein Mikroreaktor gemäß der Figuren 1 und 2 verwendet. Der Mikroreaktor ist aus Edelstahl 1.4742 gefertigt. In den Mikroreaktoreinheiten sind pro mikrostrukturierter Platte 12 Mikrokanäle vorgesehen, wobei die Mikrokanäle eine laterale Ausdehnung von (0,6 x 0,4) mm² aufweisen. Bei einer Länge der Mikrokanäle von 100 mm ergibt sich demnach für zwei mikrostrukturierte Platten ein Gesamtvolumen von 0.288 mL. Die Mikrokanäle sind ferner wie oben beschreiben durch Washcoat-Beschichten mit einem Aluminiumoxidkatalysator beschichtet. Dazu wird der Katalysator Puralox Sba200 der Firma Sasol verwendet, der folgende Eigenschaften aufweist:

| | |
|---|---|
| Spezifische innere Oberfläche (BET) | 206 m³/g |
| Schüttdichte | 0.62 mL/g |
| Al₂O₃-Gehalt | 97.1% |
| SiO₂-Gehalt | 68 ppm |
| Alkali- und Erdalkaligehalt | 20 ppm. |

Der Mikroreaktor wird durch Heizpatronen und Heizmanschetten zu Isolierung auf einer Temperatur von 470 °C gehalten, wobei sich am Katalysator eine maximale Temperatur von 450 °C einstellt.

### Vergleichsbeispiel 1

Ein Gemisch aus Methanol und Phenol in einem molaren Verhältnis 1 wird über eine Dosierpumpe bei Normaldruck und einer Flussrate von 0.1 mL/min in einen Rohrreaktor mit einem Außendurchmesser von 6 mm geleitet. Der Katalysator ist ein Aluminiumoxidkatalysator, der im Festbett vorliegt. Der Reaktor wird auf einer Temperatur von 465 °C gehalten, wobei sich am Katalysator eine maximale Temperatur von 450 °C einstellt.

Die modifizierte Verweilzeit beschreibt die Masse des Katalysators pro Volumenstrom und beträgt für den Rohrreaktor 0.71 g·h /L. Die Ergebnisse der Alkylierung von Phenol mit Methanol sind in Tabelle 1 dargestellt.

**Tabelle 1: Ergebnisse der Alkylierung von Phenol mit Methanol in einem Rohrreaktor.**

| Phenol [%] | Methanol | o-Kresol | m-/p-Kresol | 2,6-Xylenol | Weitere Xylenole | Trimethylphenole | Anisol | Wasser |
|---|---|---|---|---|---|---|---|---|
| 32.0 | 0.0 | 29.7 | 3.1 | 13.9 | 4.8 | 5.9 | 0.0 | 10.6 |

### B eispiel 1

Ein Gemisch aus Methanol und Phenol wird in einem molaren Verhältnis von 1 über eine Dosierpumpe bei Normaldruck und einer Flussrate von 0.1 mL/min über einen Vorerhitzer in einen Mikroreaktor geleitet.

Die Zusammensetzung der Reaktionsprodukte ist in Tabelle 2 wiedergegeben.

**Tabelle 2: Ergebnisse der Alkylierung von Phenol mit Methanol in einem Mikroreaktor.**

| Phenol [%] | Methanol | o-Kresol | m-/p-Kresol | 2,6-Xylenol | Weitere Xylenole | Trimethylphenole | Anisol | Wasser |
|---|---|---|---|---|---|---|---|---|
| 27.7 | 0.0 | 31.2 | 3.4 | 12.7 | 6.3 | 4.3 | 2.3 | 12.1 |

Die modifizierte Verweilzeit beträgt 7.7.10⁻² g·h/L. Im Vergleich zu oben beschriebenem Beispiel eines Rohrreaktors weist der Mikroreaktor eine um den Faktor 9.2 kleinere Verweilzeit auf. Weiterhin ist das Verhältnis o-Kresol zu Phenol für den Mikroreaktor um einen Faktor 1.21 höher als für den Rohrreaktor. Mit einem Mikroreaktor kann demnach eine bessere Raum-Zeit-Ausbeute erreicht werden.

### Vergleichsbeispiel 2

Ein Gemisch aus Ethanol und Phenol in einem molaren Verhältnis von 1 wird über eine Dosierpumpe bei Normaldruck und einer Flussrate von 0.1 mL/min in einen Rohrreaktor mit einem Außendurchmesser von 6 mm geleitet. Der Reaktor wird auf einer Temperatur von 375 °C gehalten, wobei sich am Katalysator eine maximale Temperatur von 350 °C einstellt. Die modifizierte Verweilzeit beträgt 0.68 g.h /L und die Zusammensetzung der Reaktionsprodukte ist in Tabelle 3 wieder gegeben.

**Tabelle 3: Ergebnisse der Alkylierung von Phenol mit Ethanol in einem Rohrreaktor.**

| Phenol [%] | Ethanol | 2-Ethylphenol | 3-/4-Ethylphenol | Mehrfach alkylierte Phenole | Phenetol | Wasser |
|---|---|---|---|---|---|---|
| 24.16 | 1.54 | 32.07 | 5.83 | 26.16 | 0.0 | 10.24 |

### Beispiel 2

Ein Gemisch aus Ethanol und Phenol in einem molaren Verhältnis von 1 wird über eine Dosierpumpe bei Normaldruck und einer Flussrate von 0.1 mL/min über einen Vorerhitzer in den Mikroreaktor geleitet.

Der Reaktor wird auf einer Temperatur von 425 °C gehalten, wobei sich am Katalysator eine maximale Temperatur von 405 °C einstellt. Die modifizierte Verweilzeit beträgt 7.7·10⁻² g·h/L und ist somit im Vergleich zum Rohrrektor um den Faktor 8.8 kleiner. Die Zusammensetzung der Reaktionsprodukte ist in Tabelle 4 wiedergegeben. Im Vergleich zum Rohrreaktor ergibt sich beim Mikroreaktor trotz kleinerer modifizierter Verweilzeit ein geringerer Anteil an Nebenprodukten.

**Tabelle 4: Ergebnisse der Alkylierung von Phenol mit Ethanol in einem Mikroreaktor.**

| Phenol [%] | Ethanol | 2-Ethylphenol | 3-/4-Ethylphenol | Mehrfach alkylierte Phenole | Phenetol | Wasser |
|---|---|---|---|---|---|---|
| 45.15 | 11.10 | 18.89 | 1.75 | 14.63 | 0.43 | 8.05 |

### Vergleichsbeispiel 3

Ein Gemisch aus 2-Propanol und Phenol in einem molaren Verhältnis von 1 wird über eine Dosierpumpe bei Normaldruck und einer Flussrate von 0.2 mL/min in einen Rohrreaktor mit einem Außendurchmesser von 6 mm geleitet. Der Reaktor wird auf einer Temperatur von 415 °C gehalten, wobei sich am Katalysator eine maximale Temperatur von 400 °C einstellt. Die modifizierte Verweilzeit beträgt 0.68 g·h /L. Die Zusammensetzung der Reaktionsprodukte ist in Tabelle 5 wiedergegeben.

**Tabelle 5: Ergebnisse der Alkylierung von Phenol mit 2-Propanol in einem Rohrreaktor.**

| Phenol [%] | 2-Propanol | 2-Isopropylphenol | 3-/4-Isopropylphenol | Mehrfach alkylierte Phenole | Isopropoxyphenol | Wasser |
|---|---|---|---|---|---|---|
| 56.25 | 14.44 | 10.53 | 0.79 | 0 | 6.47 | 11.56 |

### Beispiel 3

Ein Gemisch aus 2-Propanol und Phenol in einem molaren Verhältnis von 1 wird über eine Dosierpumpe bei Normaldruck und einer Flussrate von 0.1 mL/min über einen Vorerhitzer in den Mikroreaktor geleitet.

Der Mikroreaktor wird auf einer Temperatur von 370 °C gehalten, wobei sich am Katalysator eine maximale Temperatur von 385 °C einstellt. Die modifizierte Verweilzeit beträgt 8.1·10⁻² g·h/L und ist somit im Vergleich zum Rohrrektor um den Faktor 8.4 kleiner. Die Zusammensetzung der Reaktionsprodukte ist in Tabelle 6 wiedergegeben.

**Tabelle 6: Ergebnisse der Alkylierung von Phenol mit 2-Propanol in einem Mikroreaktor.**

| Phenol [%] | 2-Propanol | 2-Isopropylphenol | 3-/4-Isopropylphenol | Mehrfach alkylierte Phenole | Isopropoxyphenol | Wasser |
|---|---|---|---|---|---|---|
| 68.43 | 9.25 | 7.32 | 0.93 | 0 | 0.10 | 13.97 |

## Patentansprüche

1. Verfahren zur Herstellung von Hydroxyaromaten durch heterogene, katalytische Umsetzung von Hydroxyaromaten mit C₁-C₄-Alkanolen in einem Mikroreaktor (10) umfassend die Schritte:
a) Einleiten des Hydroxyaromaten und mindestens eines C₁-C₄-Alkanols als Edukte in mindestens eine Einlassöffnung (22) des Mikroreaktors (10) umfassend mindestens eine Mikroreaktoreinheit (18),
b) Durchleiten der Edukte durch mindestens eine Mikroreaktoreinheit (18) des Mikroreaktors (10), die eine Mehrzahl von Mikrokanälen (28) umfasst, wobei die Mikrokanäle (28) eine laterale Ausdehnung von weniger als 1 mm aufweisen und in den Mikrokanälen (28) ein heterogener Katalysator zur Umsetzung der Edukte eingebracht ist,
c) Ausleiten der hergestellten Hydroxyaromate durch mindestens eine Auslassöffnung (24) des Mikroreaktors (10).

2. Verfahren gemäß Anspruch 1, **dadurch gekennzeichnet, dass** der heterogene Katalysator einen festen Träger umfasst.

3. Verfahren gemäß Anspruch 2, **dadurch gekennzeichnet, dass** der feste Träger ein oder mehrere Komponenten aus der Gruppe bestehend aus anorganischen Oxiden, Kohlenstoff, Polymeren, Ton, einen Zeolithen oder mesoporösen Feststoffen umfasst.

4. Verfahren gemäß einem der Ansprüche 2 oder 3, **dadurch gekennzeichnet, dass** der heterogene Katalysator auf dem festen Träger eine Aktivkomponente umfasst.

5. Verfahren gemäß einem der Ansprüche 1 bis 3, **dadurch gekennzeichnet, dass** der heterogene Katalysator im Wesentlichen aus Aluminiumoxid besteht.

6. Verfahren gemäß einem der Ansprüche 1 bis 5, **dadurch gekennzeichnet, dass** die mindestens eine Mikroreaktoreinheit (18) aus mindestens zwei aufeinanderliegenden Platten (20), welche an der Oberfläche die Mikrokanäle (28) bildenden Vertiefungen (26) aufweisen, gebildet wird.

7. Verfahren gemäß Anspruch 6, **dadurch gekennzeichnet, dass** eine Mikroreaktoreinheit (18) aus 2 bis 10 aufeinanderliegenden Platten (20) gebildet wird.

8. Verfahren gemäß einem der Ansprüche 6 bis 7, **dadurch gekennzeichnet, dass** die Länge der Mikrokanäle (28) 50 mm bis 150 mm beträgt.

9. Verfahren gemäß einem der Ansprüche 1 bis 8, **dadurch gekennzeichnet, dass** der heterogene Katalysator durch Washcoat-Beschichten der Platten (20) und ggf. Imprägnierung der Washcoat-Beschichtung mit einer Aktivkomponente in die Mikrokanäle (28) eingebracht wird.

10. Verfahren gemäß einem der Ansprüche 1 bis 9, **dadurch gekennzeichnet, dass** die Edukte in flüssiger Phase, in gasförmiger Phase oder zweiphasig vorliegen.

11. Verfahren gemäß einem der Ansprüche 1 bis 10, **dadurch gekennzeichnet, dass** der Hydroxyaromat mindestens eine Phenol-Verbindung, ausgewählt aus der Gruppe bestehend aus Phenol, o-Kresol, m-Kresol, p-Kresol, Xylenolen, Trimethylphenolen, Tetramethylphenolen, Ethylphenolen, Diethylphenolen, Triethylphenolen, *iso*-Propylphenolen, Di-*iso*-propylphenolen, Tri-*iso-*propylphenolen, Tetra-*iso*-propylphenolen, *n*-Propylphenolen, Di-*n-*propylphenolen, Tri-*n*-propylphenolen, Tetra-*n*-propylphenolen, *tert-*Butylphenolen, Di-*tert*-butylphenolen, Tri-*tert*-butylphenolen und Tetra-*tert-*butylphenolen, enthält.

12. Verfahren gemäß einem der Ansprüche 1 bis 11, **dadurch gekennzeichnet, dass** als C₁-C₄-Alkanole Methanol, Ethanol, n-Propanol, Isopropanol oder Mischungen daraus eingesetzt werden.

13. Verfahren gemäß einem der Ansprüche 1 bis 12, **dadurch gekennzeichnet, dass** das Verfahren in einem Temperaturbereich von 300 bis 600 °C durchgeführt wird.

14. Verwendung der gemäß dem Verfahren nach Anspruch 1 bis 13 hergestellten Hydroxyaromaten als Lösungmittel in Drahtlacken zur Beschichtung von elektrischen und elektronischen Bauteilen und Geräten.

15. Verwendung gemäß Anspruch 14 zur Beschichtung von Kupferdrähten.
